# EUROPÄISCHE PATENTANMELDUNG

(11) **EP 2 083 025 A1**
(43) Veröffentlichungstag der Anmeldung: **29.07.2009**
(21) Anmeldenummer: 08001290.9
(22) Anmeldetag: 24.01.2008
(51) Int. Cl.: C08G 18/10, C09J 175/02, C09J 175/04, A61L 24/04

(54) **Medizinische Klebstoffe für die Chirurgie**

(71) Anmelder: Bayer MaterialScience AG, 51368 Leverkusen (DE)
(72) Erfinder: Heckroth, Heike, Dr., 51519 Odenthal (DE); Köhler, Burkhard, Dr., 34289 Zierenberg (DE); Dörr, Sebastian, Dr., 40597 Düsseldorf (DE)

(57) **Zusammenfassung**

Die vorliegende Erfindung betrifft neuartige, schnell härtende Klebstoffe auf Basis hydrophiler Polyisocyanat-Prepolymere für den Einsatz in der Chirurgie.

## Beschreibung

Die vorliegende Erfindung betrifft neuartige, schnell härtende Klebstoffe auf Basis hydrophiler Polyisocyanat-Prepolymere für den Einsatz in der Chirurgie.

In den vergangenen Jahren hat sich ein steigendes Interesse entwickelt, chirurgische Nähte durch den Einsatz von geeigneten Klebstoffen zu ersetzen oder zu unterstützen. Besonders im Bereich der plastischen Chirurgie, in der auf dünne, möglichst unsichtbare Narben Wert gelegt wird, werden vermehrt Klebstoffe eingesetzt.

Gewebekleber müssen eine Reihe von Eigenschaften haben, um bei den Chirurgen als Nahtersatz akzeptiert zu werden. Hierzu gehören eine leichte Verarbeitbarkeit und eine Eingangsviskosität, so dass der Klebstoff nicht in tiefere Gewebeschichten eindringen oder verlaufen kann. In der klassischen Chirurgie wird eine schnelle Aushärtung erfordert, wogegen in der plastischen Chirurgie eine Korrektur der Klebenaht möglich sein sollte und damit die Aushärtungsgeschwindigkeit nicht zu schnell sein darf (ca. 5 min). Die Klebeschicht sollte ein flexibler, transparenter Film sein, der nicht in einem Zeitraum kleiner drei Wochen abgebaut wird. Der Klebstoff muss biokompatibel sein und darf weder Histotoxicität noch Thrombogenität oder ein allergenes Potential besitzen.

Diverse Materialien, die als Gewebekleber eingesetzt werden, sind im Handel erhältlich. Hierzu gehören die Cyanacrylate Dermabond® (Octyl-2-Cyanoacrylat) und Histoacryl Blue® (Butyl-Cyanoacrylat). Die schnelle Aushärtezeit, sowie die Sprödigkeit der Klebestelle limitieren jedoch den Einsatz. Durch die schlechte Bioabbaubarkeit sind Cyanacrylate nur für äußerliche chirurgische Nähte geeignet.

Als Alternative zu den Cyanacrylaten stehen biologische Klebstoffe wie peptidbasierte Substanzen (BioGlue^{®} oder Fibrinkleber (Tissucol) zur Verfügung. Neben den hohen Kosten zeichnen sich Fibrinkleber durch eine relative schwache Klebstärke und einen schnellen Abbau aus, so dass dieser nur bei kleineren Schnitten auf nicht gespannter Haut anwendbar ist.

Isocyanat-haltige Klebstoffe basieren alle auf einem aromatischen Diisocyanat und einem hydrophilen Polyol, wobei bevorzugt die Isocyanate TDI und MDI eingesetzt werden (US 20030135238, US 20050129733). Beide können, um die Reaktivität zu erhöhen, Elektronen ziehende Substituenten tragen (WO-A 03/9323).

Schwierigkeiten waren bislang die geringe mechanische Stärke (US 5,156,613), zu langsame Aushärtungsgeschwindigkeit (US 4,806,614), die zu schnelle Bioabbaubarkeit (US 6,123,667) sowie unkontrollierte Schwellung (US 6,265,016).

Gemäß Patent US 20030135238 stellen ausschließlich Polyurethan-Prepolymere mit trifunktioneller oder verzweigter Struktur, die dazu in der Lage sind, Hydrogele zu bilden, geeignete Klebstoffe dar. Der Klebstoff muß dabei in der Lage sein, eine kovalente Bindung zum Gewebe auszubilden. US 20030135238 und US 20050129733 beschreiben die Synthese trifunktioneller, Ethylenoxid reicher TDI-und IPDI- (US 20030135238) basierter Prepolymere, die mit Wasser oder mit Gewebeflüssigkeiten zum Hydrogel reagieren. Ausreichend schnelle Aushärtung wurde bislang nur bei Verwendung aromatischer Isocyanate erreicht, welche jedoch unter Schaumbildung reagieren. Dadurch kommt es zu einem Eindringen des Klebstoffes in die Wunde und damit zum Auseinanderdrücken der Wundränder, was eine schlechtere Heilung mit verstärkter Narbenbildung mit sich bringt. Durch die Schaumbildung ist zudem die mechanische Festigkeit sowie die Haftung der Klebeschicht herabgesetzt. Aufgrund der hohen Reaktivität der Prepolymere kommt es zudem zu einer Reaktion der Isocyanatreste mit dem Gewebe, wobei oftmals eine Denaturierung, erkennbar durch Weißfärbung des Gewebes, auftritt.

Als Ersatz der aromatischen Isocyanate wurde Lysindiisocyanat untersucht, welches jedoch aufgrund seiner geringen Reaktivität nicht oder nur langsam mit Gewebe reagiert (US 20030135238).

Aliphatische Isocyanate wurden, um die Reaktivität zu erhöhen, fluoriert (US 5,173,301), wodurch es jedoch spontan zu einer Selbstpolymerisation des Isocyanates kam.

EP-A 0 482 467 beschreibt die Synthese eines chirurgischen Klebstoffes basierend auf einem aliphatischen Isocyanat (bevorzugt HDI) und einem Polyethylenglycol (Carbowax 400). Die Aushärtung erfolgt bei Zugabe von 80 bis 100 % Wasser und einem Metallcarboxylat (Kaliumoctoat) als Katalysator wobei sich ein Schaum bildet, der mit Silikonöl stabilisiert wird.

Auf aliphatischen Isocyanaten basierte Systeme zeigen eine nur ungenügende Reaktivität und damit eine zu langsame Aushärtungszeit. Durch den Einsatz von Metallkatalysatoren konnte, wie in EP-A 0 482 467 beschrieben, zwar die Reaktionsgeschwindigkeit erhöht werden, doch kam es zur Bildung eines Schaums, mit den oben beschriebenen Problemen.

Eine grundsätzliche Eignung von Asparaginsäureestern zur Vernetzung von Prepolymeren ist im Stand der Technik im Rahmen von Oberflächenbeschichtungen durchaus bekannt und beispielsweise in EP-A 1 081 171 oder DE-A 102 46 708 beschrieben.

Die nicht vorveröffentlichte Europäische Patentanmeldung Nr. 07021764.1 beschreibt bereits Wundkleber auf Basis einer Kombination hydrophiler Polyisocyanat-Prepolymere und Aspartaten als Härter. Diese Systeme sind jedoch zum Teil schwierig zu dosieren und zu applizieren, da relativ zum auszuhärtenden Prepolymer nur wenig Aspartat notwendig ist. Dies kann durch eine Vorverlängerung des Aspartats mit NCO-Prepolymer verbessert werden.

Es wurde nun allerdings gefunden, dass dieses Problem statt oder zusätzlich zum Einsatz des vorverlängerten Asparats durch spezielle Füllstoffe erreicht bzw. unterstützt werden kann.

Gegenstand der vorliegenden Erfindung sind daher Klebstoffsysteme umfassend
A) isocyanatfunktionelle Prepolymere erhältlich aus
   A1) aliphatischen Isocyanaten und
   A2) Polyolen mit zahlenmittleren Molekulargewichten von ≥ 400 g/mol und mittleren OH-Funktionalitäten von 2 bis 6
   und
B) eine Härterkomponente umfassend
   B1)aminofunktionelle Asparaginsäureester der allgemeinen Formel (I) 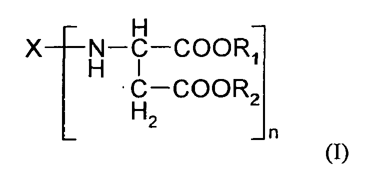 wobei
      X ein n-wertiger organischer Rest ist, der durch Entfernung der primären Aminogruppen eines n-wertigen Amins erhalten wird,
      R₁, R₂ gleiche oder verschiedene organische Reste sind, die keinen Zerewitinoff-aktiven Wasserstoff aufweisen und
      n eine ganze Zahl von mindestens 2 ist
      und
   B2)organische Füllstoffe, die eine nach DIN 53019 gemessenen Viskosität bei 23°C im Bereich von 10 bis 6000 mPas aufweisen
   und
C) gegebenenfalls Umsetzungsprodukte von isocyanatfunktionellen Prepolymeren gemäß der Definition der Komponente A) mit Asparaginsäureestern gemäß Komponente B1) und/oder organischen Füllstoffen gemäß Komponente B2).

Zur Definition von Zerewitinoff-aktivem Wasserstoff wird auf Römpp Chemie Lexikon, Georg Thieme Verlag Stuttgart verwiesen. Bevorzugt werden unter Gruppen mit Zerewitinoff-aktivem Wasserstoff OH, NH oder SH verstanden.

Unter Gewebe im Rahmen der vorliegenden Erfindung werden Zellverbände verstanden, die aus Zellen gleicher Gestalt und Leistung bestehen wie Deckgewebe (Haut), Epithelgewebe, Myocard, Binde- oder Stützgewebe, Muskeln, Nerven und Knorpel zu verstehen. Hierzu gehören unter anderem auch alle aus Zellverbänden aufgebaute Organe wie Leber, Niere, Lunge, Herz etc.

Die in A) eingesetzten isocyanatfunktionellen Prepolymere sind durch Umsetzung von Isocyanaten mit hydroxyfunktionellen Polyolen gegebenenfalls unter Zusatz von Katalysatoren sowie Hilfs- und Zusatzstoffen erhältlich.

In A1) können als Isocyanate beispielsweise monomere aliphatische oder cycloaliphatische Di- oder Triisocyanate wie 1,4-Butylendiisocyanat (BDI), 1,6-Hexamethylendiisocyanat (HDI), Isophorondiisocyanat (IPDI), 2,2,4- und/oder 2,4,4-Trimethylhexamethylendiisocyanat, die isomeren Bis-(4,4'-isocyanatocyclohexyl)-methane oder deren Mischungen beliebigen Isomerengehalts, 1,4-Cyclohexylendiisocyanat, 4-Isocyanatomethyl-1,8-octandiisocyanat (Nonan-triisocyanat), sowie Alkyl-2,6-diisocyanatohexanoate (Lysindiisocyanat) mit C1-C8-Alkylgruppen eingesetzt werden.

Neben den vorstehend genannten monomeren Isocyanaten können auch deren höhermolekulare Folgeprodukte mit Uretdion-, Isocyanurat-, Urethan-, Allophanat-, Biuret-, Iminooxadiazindion- oder Oxadiazintrionstruktur sowie deren Mischungen eingesetzt werden.

Bevorzugt werden in A1) Isocyanate der vorstehend genannten Art mit ausschließlich aliphatisch oder cycloaliphatisch gebundenen Isocyanatgruppen oder deren Mischungen eingesetzt.

Die in A1) eingesetzten Isocyanate oder Isocyanatmischungen haben bevorzugt eine mittlere NCO-Funktionalität von 2 bis 4, besonders bevorzugt 2 bis 2,6 und ganz besonders bevorzugt 2 bis 2,4.

In einer besonders bevorzugten Ausführungsform wird in A1) Hexamethylendiisocyanat eingesetzt.

Zum Prepolymeraufbau können in A2) grundsätzliche alle dem Fachmann an sich bekannten Polyhydroxyverbindungen mit 2 oder mehr OH-Funktionen pro Molekül eingesetzt werden. Dies können beispielsweise Polyesterpolyole, Polyacrylatpolyole, Polyurethanpolyole, Polycarbonatpolyole, Polyetherpolyole, Polyesterpolacrylatpolyole, Polyurethanpolyacryl-atpolyole, Polyurethanpolyesterpolyole, Polyurethanpolyetherpolyole, Polyurethanpolycarbonat-polyole, Polyesterpolycarbonatpolyole oder deren beliebige Mischungen untereinander sein.

### Die in A2) eingesetzten Polyole haben bevorzugt eine mittlere OH-Funktionalität von 3 bis 4

Die in A2) eingesetzten Polyole haben ferner bevorzugt ein zahlenmittleres Molekulargewicht von 400 bis 20000 g/mol, besonders bevorzugt 2000 bis 10000 g/mol und ganz besonders bevorzugt 4000 bis 8500.

Polyetherpolyole sind bevorzugt Polyalkylenoxid-Polyether auf Basis von Ethylenoxid und gegebenenfalls Propylenoxid.

Diese Polyetherpolyole basieren bevorzugt auf di- oder höherfunktionellen Startermolekülen wie zwei- oder höherfunktionellen Alkoholen oder Aminen.

Beispiele solcher Starter sind Wasser (als Diol aufgefasst), Ethylenglykol, Propylenglykol, Butylenglykol, Glycerin, TMP, Sorbit, Pentaerythrit, Triethanolamin, Ammoniak oder Ethylendiamin.

Bevorzugte Polyalkylenoxid-Polyether entsprechen denen der vorstehend genannten Art und weisen Gehalt an Ethylenoxid-basierten Einheiten von 50 bis 100 %, bevorzugt 60 bis 90 % bezogen auf die insgesamt enthaltene Mengen an Alkylenoxideinheiten auf.

Bevorzugte Polyesterpolyol sind die an sich bekannten Polykondensate aus Di- sowie gegebenenfalls Tri- und Tetraolen und Di- sowie gegebenenfalls Tri- und Tetracarbonsäuren oder Hydroxycarbonsäuren oder Lactonen. Anstelle der freien Polycarbonsäuren können auch die entsprechenden Polycarbonsäureanhydride oder entsprechende Polycarbonsäureester von niederen Alkoholen zur Herstellung der Polyester verwendet werden.

Beispiele für geeignete Diole sind Ethylenglykol, Butylenglykol, Diethylenglykol, Triethylenglykol, Polyalkylenglykole wie Polyethylenglykol, weiterhin 1,2-Propandiol, 1,3-Propandiol, Butandiol(1,3), Butandiol(1,4), Hexandiol(1,6) und Isomere, Neopentylglykol oder Hydroxypivalinsäureneopentylglykolester, wobei Hexandiol(1,6) und Isomere, Butandiol(1,4), Neopentylglykol und Hydroxypivalinsäureneopentylglykolester bevorzugt sind. Daneben können auch Polyole wie Trimethylolpropan, Glycerin, Erythrit, Pentaerythrit, Trimetylolbenzol oder Trishydroxyethylisocyanurat eingesetzt werden.

Als Dicarbonsäuren können Phthalsäure, Isophthalsäure, Terephthalsäure, Tetrahydrophthalsäure, Hexahydrophthalsäure, Cyclohexandicarbonsäure, Adipinsäure, Azelainsäure, Sebacinsäure, Glutarsäure, Tetrachlorphthalsäure, Maleinsäure, Fumarsäure, Itaconsäure, Malonsäure, Korksäure, 2-Methylbemsteinsäure, 3,3-Diethylglutarsäure und/oder 2,2-Dimethylbernsteinsäure eingesetzt werden. Als Säurequelle können auch die entsprechenden Anhydride verwendet werden.

Sofern die mittlere Funktionalität des zu veresternden Polyols > als 2 ist, können zusätzlich auch Monocarbonsäuren, wie Benzoesäure und Hexancarbonsäure mit verwendet werden.

Bevorzugte Säuren sind aliphatische oder aromatische Säuren der vorstehend genannten Art. Besonders bevorzugt sind Adipinsäure, Isophthalsäure und Phthalsäure.

Hydroxycarbonsäuren, die als Reaktionsteilnehmer bei der Herstellung eines Polyesterpolyols mit endständigen Hydroxylgruppen mitverwendet werden können, sind beispielsweise Hydroxycapronsäure, Hydroxybuttersäure, Hydroxydecansäure, Hydroxystearinsäure und dergleichen. Geeignete Lactone sind Caprolacton, Butyrolacton und Homologe. Bevorzugt ist Caprolacton.

Ebenfalls können hydroxylgruppenaufweisende Polycarbonate, bevorzugt Polycarbonatdiole, mit zahlenmittleren Molekulargewichten Mₙ von 400 bis 8000 g/mol, bevorzugt 600 bis 3000 g/mol eingesetzt werden. Diese sind durch Reaktion von Kohlensäurederivaten, wie Diphenylcarbonat, Dimethylcarbonat oder Phosgen, mit Polyolen, bevorzugt Diolen, erhältlich.

Beispiele derartiger Diole sind Ethylenglykol, 1,2- und 1,3-Propandiol, 1,3- und 1,4-Butandiol, 1,6-Hexandiol, 1,8-Octandiol, Neopentylglykol, 1,4-Bishydroxymethylcyclohexan, 2-Methyl-1,3-propandiol, 2,2,4-Trimethylpentandiol-1,3, Dipropylenglykol, Polypropylenglykole, Dibutylenglykol, Polybutylenglykole, Bisphenol A und lactonmodifizierte Diole der vorstehend genannten Art in Frage.

Bevorzugt werden zum Prepolymeraufbau Polyetherpolyole der vorstehend genannten Art eingesetzt.

Zur Herstellung des Prepolymers werden die Verbindungen der Komponente A1) mit denen der Komponente A2) bei einem NCO/OH-Verhältnis von bevorzugt 4:1 bis 12:1, besonders bevorzugt 8:1 umgesetzt und anschließend der Anteil an nicht umgesetzten Verbindungen der Komponente A1) mittels geeigneter Methoden abgetrennt. Üblicherweise wird hierfür die Dünnschichtdestillation verwendet, wobei restmonomerenarme Produkte mit Restmonomergehalten von weniger als 1 Gew.-%, bevorzugt weniger als 0,5 Gew.-%, ganz besonders bevorzugt weniger als 0,1 Gew.-% erhalten werden.

Gegebenenfalls können während der Herstellung Stabilisatoren wie Benzoylchlorid, Isophthaloylchlorid, Dibutylphosphat, 3-Chlorpropionsäure oder Methyltosylat zugesetzt werden.

Die Reaktionstemperatur beträgt dabei 20 bis 120°C, bevorzugt 60 bis 100°C.

### Bevorzugt sind in Formel (I):

- R₁, R₂: gleiche oder verschiedene gegebenenfalls verzweigte oder cyclische organische Reste, die keinen Zerewitinoff-aktiven Wasserstoff aufweisen, mit 1 bis 20, bevorzugt 1 bis 10 Kohlenstoffatomen, besonders bevorzugt Methyl- oder Ethylgruppen, ,
- n: eine ganze Zahl von 2 bis 4 und
- X: ein n-wertiger organischer gegebenenfalls verzweigter oder cyclischer organischer Rest mit 2 bis 20, bevorzugt 5 bis 10 Kohlenstoffatomen, der durch Entfernung der primären Aminogruppen eines n-wertigen primären Amins erhalten wird.

Selbstverständlich können auch Gemische von mehreren Asparaginsäureestern verwendet werden, so dass n in Formel (I) auch einen nicht ganzzahligen Mittelwert darstellen kann.

Die Herstellung der aminofunktionellen Polyasparaginsäureester B1) erfolgt in bekannter Weise durch Umsetzung der entsprechenden primären mindestens difunktionellen Amine X(NH₂)ₙ mit Malein- oder Fumarsäureestern der allgemeinen Formel

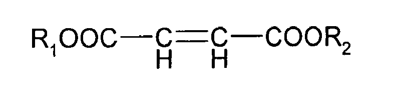

Bevorzugte Malein- oder Fumarsäureester sind Maleinsäuredimethylester, Maleinsäurediethylester, Maleinsäuredibutylester und die entsprechenden Fumarsäureester.

Bevorzugte primäre mindestens difunktionelle Amine X(NH₂)ₙ sind Ethylendiamin, 1,2-Diaminopropan, 1,4-Diaminobutan, 1,3-Diaminopentan, 1,5-Diaminopentan, 2-Methyl-1,5-Diaminopentan, 1,6-Diaminohexan, 2,5-Diamino-2,5-dimethylhexan, 2,2,4- und/oder 2,4,4-Trimethyl-1,6-diaminohexan, 1,11-Diaminoundecan, 1,12-Diaminododecan, 1-Amino-3,3,5-trimethyl-5-aminomethyl-cyclohexan, 2,4-und/oder 2,6-Hexahydrotoluylendiamin, 2,4'-und/oder 4,4'-Diamino-dicyclohexylmethan, 3,3'-Dimethyl-4,4'-diamino-dicyclohexyl-methan, 2,4,4'-Triamino-5-methyl-dicyclohexylmethan und Polyetheramine mit aliphatisch gebundenen primären Aminogruppen mit einem zahlenmittleren Molekulargewicht Mₙ von 148 bis 6000 g/mol.

Besonders bevorzugte primäre mindestens difunktionelle Amine sind 1,3-Diaminopentan, 1,5-Diaminopentan, 2-Methyl-1,5-diaminopentan, 1,6-Diaminohexan, 1,13-Diamino-4,7,10-trioxatridecan. Ganz besonders bevorzugt ist 2-Methyl-1,5-diaminopentan.

In einer bevorzugten Ausführungsform der Erfindung sind R₁ = R₂ = Ethyl, wobei X auf 2-Methyl-1,5-diaminopentan, als n-wertigem Amin basiert.

Die Herstellung der aminofunktionellen Asparaginsäureester B1) aus den genannten Ausgangsmaterialien erfolgt nach DE-A 69 311 633 bevorzugt innerhalb des Temperaturbereichs von 0 bis 100 °C, wobei die Ausgangsmaterialien in solchen Mengenverhältnissen eingesetzt werden, dass auf jede primäre Aminogruppe mindestens eine, vorzugsweise genau eine olefinische Doppelbindung entfällt, wobei im Anschluss an die Umsetzung gegebenenfalls im Überschuss eingesetzte Ausgangsmaterialien destillativ abgetrennt werden können. Die Umsetzung kann in Substanz oder in Gegenwart geeigneter Lösungsmittel wie Methanol, Ethanol, Propanol oder Dioxan oder Gemischen derartiger Lösungsmittel erfolgen.

Die in B2) eingesetzten organischen flüssigen Füllstoffe sind bevorzugt gemäß Zytotoxizitätsmessung nach ISO 10993 nicht zytotoxisch.

Als organische Füllstoffe können unter anderem Polyethylenglykole wie PEG 200 bis PEG 600, deren Mono- bzw. Dialkylether wie PEG 500 Dimethylether, Polyether- und Polyesterpolyole, Polyester wie Ultramoll, Lanxess GmbH, DE sowie Glycerin und seine Derivate wie Triacetin Lanxess GmbH, DE eingesetzt werden, sofern sie der anspruchsgemäßen Viskosität genügen.

Bevorzugt handelt es sich bei den organischen Füllstoffen der Komponente B2) um hydroxy- oder aminofunktionelle, bevorzugt rein hydroxyfunktionelle Verbindungen. Besonders bevorzugt sind Polyole. Bevorzugte Polyole sind Polyether und/oder Polyesterpolyole, besonders bevorzugt Polyetherpolyole.

Die bevorzugten organischen Füllstoffe der Komponente B2) besitzen bevorzugt mittlere OH-Funktionalitäten von 1,5 bis 3, besonders bevorzugt 1,8 bis 2,2, ganz besonders bevorzugt 2,0.

Die bevorzugten organischen Füllstoffe der Komponente B2) besitzen bevorzugt sich wiederholende von Ethylenoxid abgeleitete Einheiten.

Die Viskosität der organischen Füllstoffe der Komponente B2) beträgt bevorzugt 50 bis 4000 mPas bei 23°C gemessen nach DIN 53019.

In einer bevorzugten Ausführungsform der Erfindung werden als organische Füllstoffe der Komponente B2) Polyethylenglycole eingesetzt. Diese haben bevorzugt ein zahlenmittleres Molekulargewicht von 100 bis 1000 g/mol, besonders bevorzugt 200 bis 400 g/mol.

Das Gewichtsverhältnis von B1) zu B2) beträgt 1:0 bis 1:20 , bevorzugt 1:0 bis 1:12.

Das Gewichtsverhältnis der Komponente B2 bezogen auf die Gesamtmenge der Mischung aus B1, B2 und A liegt im Bereich von 0 bis 100 %, bevorzugt 0 bis 60 %.

Um das mittlere Equivalentgewicht der insgesamt zur Prepolymervernetzung eingesetzten Verbindungen bezogen auf die NCO-reaktiven Gruppen weiter zu reduzieren, ist es möglich zusätzlich zu den in B1) und B2) eingesetzten Verbindungen auch die amino- oder hydroxyfunktionellen Umsetzungsprodukte isocyanatfunktioneller Prepolymere mit Asparaginsäureestern und/oder organischen Füllstoffen B2), sofern diese amino- oder hydroxyfunktionell sind, in einer separaten Vorreaktion herzustellen und dann als höhermolekulare Härterkomponente C) einzusetzen.

Bevorzugt werden bei der Vorverlängerung Verhältnisse von isocyanatreaktiven Gruppen zu Isocyanatgruppen von 50 zu 1 bis 1,5 zu 1 eingesetzt, besonders bevorzugt 15 zu 1 bis 4 zu 1.

Das dafür einzusetzende isocyanatfunktionelle Prepolymer kann dabei demjenigen der Komponente A) entsprechen oder aber auch abweichend aus den Komponenten, wie sie als mögliche Bestandteile der isocyanatfunktionellen Prepolymere im Rahmen dieser Anmeldung gelistet sind, aufgebaut sein.

Vorteil dieses Modifizierung durch Vorverlängerung ist, dass das Equivalentgewicht und Equivalentvolumen der Härterkomponente in deutlichen Grenzen modifizierbar ist. Dadurch können zur Applikation kommerziell verfügbare 2-Kammerdosiersysteme eingesetzt werden, um ein Klebesystem zu erhalten, das bei bestehenden Verhältnissen der Kammervolumina in das gewünschte Verhältnis von NCO-reaktiven Gruppen zu NCO-Gruppen eingestellt werden.

Die erfindungsgemäßen 2-komponentigen Klebstoffsysteme werden durch Mischung des Prepolymers mit der Härterkomponente B) bzw. C) erhalten. Das Verhältnis von NCO-reaktiven NH-Gruppen zu freien NCO-Gruppen beträgt bevorzugt 1:1,5 bis 1:1, besonders bevorzugt 1:1.

Die erfindungsgemäßen 2-komponentigen Klebstoffsysteme besitzen unmittelbar nach Vermischung der Einzelkomponenten miteinander eine Scherviskosität bei 23°C von bevorzugt 1000 bis 10000 mPas, besonders bevorzugt 1000 bis 8000 mPas und ganz besonders bevorzugt 1000 bis 4000 mPas.

Die Geschwindigkeit bei 23°C bis eine vollständige Vernetzung und Aushärtung des Klebestoffs erreicht ist, beträgt typischerweise 30 s bis 10 min, bevorzugt 1 min bis 8 min, besonders bevorzugt 1 min bis 5 min.

Ein weiterer Gegenstand der Erfindung sind die aus den erfindungsgemäßen Klebstoffsystemen erhältlichen Klebfilme sowie daraus hergestellte Verbundteile.

In einer bevorzugten Ausführungsform werden die erfindungsgemäßen 2-komponentigen Klebstoffsysteme als Gewebekleber für den Verschluss von Wunden in menschlichen oder tierischen Zellverbänden verwendet, so dass auf ein Klammern oder Nähen zum Verschließen weitestgehend verzichtet werden kann.

Der erfindungsgemäße Gewebekleber kann so wohl in vivo als auch in vitro angewendet werden, wobei die in vivo Anwendung beispielsweise zur Wundbehandlung nach Unfällen oder Operationen bevorzugt ist.

Daher ist auch ein Gegenstand der vorliegenden Erfindung ein Verfahren zum Verschließen oder Verbinden von Zellgeweben, **dadurch gekennzeichnet, dass** die erfindungsgemäßen 2-komponentige Klebstoffsysteme eingesetzt werden.

Ebenfalls Gegenstand der Erfindung ist ferner die Verwendung solcher 2-komponentigen Klebstoffsysteme zur Herstellung eines Mittels zum Verschließen oder Verbinden von Zellgeweben, so wie die zur Applikation notwendigen 2-Kammerdosiersysteme umfassend die erfindungswesentlichen Komponenten des Klebstoffsystems.

### Beispiele:

Sofern nicht abweichend angegeben beziehen sich alle Prozentangaben auf das Gewicht.

Zur Verklebung *in vitro* wurde als Gewebe Rind- oder Schweinefleisch verwendet. Es wurden jeweils zwei Stücke Fleisch (1 = 4 cm, h = 0.3 cm, b = 1 cm) an den Enden 1 cm weit mit dem Kleber bestrichen und überlappend geklebt. Die Stabilität der Klebeschicht wurde jeweils durch Zug überprüft.

PEG = Polyethylenglykol

### Beispiel 1 (Prepolymer A)

465 g HDI und 2.35 g Benzoylchlorid wurden in einem 1 1 Vierhalskolben vorgelegt. Innerhalb von 2h wurden bei 80°C 931.8 g eines Polyethers mit einem Ethylenoxidgehalt von 63% und einem Propylenoxidgehalt von 37% (jeweils bezogen auf dem gesamten Alkylenoxidgehalt) gestartet auf TMP (3-funktionell) hinzugefügt und rührte 1h nach. Anschließend wurde durch Dünnschichtdestillation bei 130°C und 0,1 Torr das überschüssige HDI abdestilliert. Man erhielt 980 g (71 %) des Präpolymers mit einem NCO-Gehalt von 2,53%. Der Restmonomerengehalt betrug < 0,03 % HDI.

### Beispiel 2 (Aspartat B)

Zu 2 Mol Diethylmaleat wurde unter Stickstoffatmosphäre langsam 1 Mol 2-Methyl-1,5-diaminopentan getropft, so dass die Reaktionstemperatur 60°C nicht überschritt. Anschließend wurde so lange auf 60°C erwärmt, bis kein Diethylmaleat mehr im Reaktionsgemisch nachweisbar war. Das Produkt wurde durch Destillation gereinigt.

### Beispiel 2a (Aspartat-Komponente partiell mit Isocyanat-funktionellem Prepolymer vorverlängert)

1000 g HDI (Hexamethylendiisocyanat), 1 g Benzoylchlorid und 1 g *para-*Toluonsulfonsäuremethylester wurden in einem 4 1 Vierhalskolben unter Rühren vorgelegt. Innerhalb von 3 Stunden fügte man bei 80°C 1000 g eines difunktionellen Polypropylenglykol-Polyethers mit einem mittleren Molekulargewicht von 2000 g/mol hinzu und rührte 1h nach. Anschließend wurde durch Dünnschichtdestillation bei 130°C und 0,1 Torr das überschüssige HDI abdestilliert. Das erhaltene Prepolymer hat einen NCO-Gehalt von 3,7%.

200 g des Prepolymers wurden in einen 1 1 Vierhalskolben unter Rühren bei Raumtemperatur zu 291 g des Aspartats B) aus 2,-Methyl-1,5-diaminopentan dosiert. Es wurde zwei Stunden nachgerührt, bis IR-spektroskopisch keine Isocyanatgruppen mehr nachweisbar waren. Das erhaltene Produkt hatte eine Viskosität von 3740 mPas und ein NH-Equivalentgewicht von 460 g/eq.

### Beispiele zur Klebung von Gewebe:

### Beispiel 3a in vitro Klebung von Muskelgewebe

1 g des vorverlängerten Aspartats aus Beispiel 2a wurde in die 1 ml fassende Kammer eines käuflichen 2-K- Spritzensystems gefüllt. Die zweite, 4 ml fassende Kammer, wurde mit 4 g des Prepolymers A gefüllt. Die Komponenten wurden durch Herunterdrücken der Stempel durch einen aufgesetzten Statikmischer mit entsprechendem Applikator gepresst und auf das Gewebe dünn aufgetragen. Es hat innerhalb von 2 min eine starke Klebung stattgefunden. Durch Zug konnten die Gewebestücke nicht ohne Faserriss voneinander getrennt werden. Bei Auftragung auf die Oberfläche des Gewebes fand eine vollständige Aushärtung unter Bildung eines durchsichtigen Filmes innerhalb von 3 Minuten statt.

### Beispiel 3b in vitro Klebung von Haut

Die Mischung aus Beispiel 3a wurde auf eine 2x2 cm große Fläche auf dem rasierten Rücken eines Hausschweins aufgetragen und das Klebeverhalten über den Zeitraum von einer Woche beobachtet. Eine Aushärtung zu einem durchsichtigen Film hatte innerhalb von 3 min stattgefunden. Der Film zeigte auch nach einer Woche keine Abschälung oder Veränderung.

### Beispiel 4a in vitro Klebung von Muskelgewebe

0,45 g PEG 200 (60 mPas/20°C) wurden mit 0,55 g Aspartat B gut vermischt und mit 4 g des Prepolymers A wie in Beispiel 3a beschrieben appliziert. Eine Aushärtung mit einer damit verbundenen starken Klebung hatte innerhalb von 2 min stattgefunden. Durch Zug konnten die Gewebestücke nicht ohne Faserriss voneinander getrennt werden. Bei Auftragung auf die Oberfläche des Gewebes fand eine vollständige Aushärtung unter Bildung eines durchsichtigen Filmes innerhalb von 3 Minuten statt.

### Beispiel 4b in vitro Klebung von Haut

Die Mischung aus Beispiel 4a wurde auf eine 2x2 cm große Fläche auf dem rasierten Rücken eines Hausschweins aufgetragen und das Klebeverhalten über den Zeitraum von einer Woche beobachtet. Eine Aushärtung zu einem durchsichtigen Film hatte innerhalb von 3 min stattgefunden. Der Film zeigte auch nach einer Woche keine Abschälung oder Veränderung.

### Vergleichsbeispiel 5 in vitro Klebung von Haut

0,55 g Aspartat B wurde mit 4 g des Prepolymers A gut vermischt und auf eine 2x2 cm große Fläche auf dem rasierten Rücken eines Hausschweins aufgetragen. Das Klebeverhalten wurde über den Zeitraum von einer Woche beobachtet. Eine Aushärtung zu einem durchsichtigen Film hatte innerhalb von 3 min stattgefunden. Der Film zeigte nach 4 Tagen eine leichte Abschälung an den Rändern. Bei der entsprechenden *in vitro*-Verklebung von Gewebe hat eine Aushärtung mit starker Klebung innerhalb von 2 min stattgefunden. Durch Zug konnten die Gewebestücke nicht ohne Faserriss voneinander getrennt werden.

### Beispiel 6 in vitro Klebung von Muskelgewebe

4 g des Prepolymers A wurden mit einer Mischung aus 6 g PEG 200 (60 mPas/20°C) und 0,55 g Aspartat B in einem Becher gut verrührt. Die Reaktionsmischung wurde unmittelbar danach auf das zu klebende Gewebe dünn aufgetragen. Eine Aushärtung mit einer damit verbundenen starken Klebung hatte innerhalb von 2 min stattgefunden. Durch Zug konnten die Gewebestücke nicht ohne Faserriss voneinander getrennt werden. Bei Auftragung auf die Oberfläche des Gewebes fand eine vollständige Aushärtung unter Bildung eines durchsichtigen Filmes innerhalb von 3 Minuten statt.

### Beispiel 7 in vitro Klebung von Muskelgewebe

4 g des Prepolymers A wurden mit einer Mischung aus 12 g PEG 200 (60 mPas/20°C) und 0,55 g Aspartat B in einem Becher gut verrührt und auf das zu klebende Gewebe dünn aufgetragen. Es hat nach 2 min eine mittelmäßige Klebung stattgefunden. Durch Zug konnten die Gewebestücke unbeschädigt voneinander getrennt werden.

### Beispiel 8 in vitro Klebung von Muskelgewebe

4 g des Prepolymers A wurden mit einer Mischung aus 18 g PEG 200 (60 mPas/20°C) und 0,55 g Aspartat B in einem Becher gut verrührt und auf das zu klebende Gewebe dünn aufgetragen. Es hat nach 2 min lediglich eine schwache Adhäsion zwischen den beiden Gewebestücken stattgefunden.

### Beispiel 9 in vitro Klebung von Muskelgewebe

0,45 g PEG 400 (120 mPas/20°C) wurden mit 0,55 g Aspartat B gut vermischt und mit 4 g des Prepolymers A wie in Beispiel 3a beschrieben appliziert. Es hat nach 2 min eine gute Klebung stattgefunden. Durch Zug konnten die Gewebestücke nicht ohne Faserriss voneinander getrennt werden. Bei Auftragung auf die Oberfläche des Gewebes fand eine vollständige Aushärtung unter Bildung eines durchsichtigen Filmes innerhalb von 10 Minuten statt.

### Beispiel 10 in vitro Klebung von Muskelgewebe

4 g des Prepolymers A wurden mit einer Mischung aus 3,45 g PEG 400 (120 mPas/20°C) und 0,55 g Aspartat B in einem Becher gut verrührt. und auf das zu klebende Gewebe dünn aufgetragen. Es hat nach 2 min eine mittelmäßige Klebung stattgefunden. Durch Zug konnten die Gewebestücke unbeschädigt voneinander getrennt werden. Bei Auftragung auf die Oberfläche des Gewebes fand eine vollständige Aushärtung unter Bildung eines durchsichtigen Filmes innerhalb von 10 Minuten statt.

### Beispiel 11 in vitro Klebung von Muskelgewebe

0,45 g PEG 600 (180 mPas/25°C) wurden mit 0,55 g Aspartat B gut vermischt und mit 4 g des Prepolymers A wie in Beispiel 3a beschrieben appliziert Es hat nach 2 min eine gute Klebung stattgefunden. Durch Zug konnten die Gewebestücke mit leichter Faserschädigung voneinander getrennt werden. Bei Auftragung auf die Oberfläche des Gewebes fand eine vollständige Aushärtung unter Bildung eines durchsichtigen Filmes innerhalb von 10 Minuten statt.

### Beispiel 12 in vitro Klebung von Muskelgewebe

4 g des Prepolymers A wurden mit einer Mischung aus 3,45 g PEG 600 (180 mPas/25°C) und 0,55 g Aspartat B in einem Becher gut verrührt und auf das zu klebende Gewebe dünn aufgetragen. Es hat nach 2 min eine mittelmäßige Klebung stattgefunden. Durch Zug konnten die Gewebestücke ohne Faserschädigung voneinander getrennt werden. Bei Auftragung auf die Oberfläche des Gewebes fand eine vollständige Aushärtung unter Bildung eines durchsichtigen Filmes innerhalb von 10 Minuten statt.

### Beispiel 13 in vitro Klebung von Muskelgewebe

4 g des Prepolymers A wurden mit einer Mischung aus 6 g PEG 600 (180 mPas/25°C) und 0,55 g Aspartat B in einem Becher gut verrührt und auf das zu klebende Gewebe dünn aufgetragen. Es hat nach 3 min eine leichte Klebung stattgefunden. Durch Zug konnten die Gewebestücke ohne Faserschädigung voneinander getrennt werden. Bei Auftragung auf die Oberfläche des Gewebes fand eine vollständige Aushärtung unter Bildung eines durchsichtigen Filmes innerhalb von 10 Minuten statt.

### Beispiel 14 in vitro Klebung von Muskelgewebe

4 g des Prepolymers A wurden mit einer Mischung aus 0,55 g Aspartat B und 3,45 g eines Polyethers des Molekulargewichts 218 mit einem Propylenoxidanteil von 65 % und der Funktionalität 2 (80 mPas/20°C) in einem Becher gut verrührt und auf das zu klebende Gewebe dünn aufgetragen. Es hat nach 2 min eine gute Klebung stattgefunden. Durch Zug konnten die Gewebestücke nicht ohne Faserschädigung voneinander getrennt werden. Bei Auftragung auf die Oberfläche des Gewebes oder auf Haut fand in einem Zeitraum von 3 Minuten eine vollständige Aushärtung unter Bildung eines durchsichtigen Filmes statt.

### Vergleichsbeispiele zur in vitro Klebung von Muskelgewebe:

### Beispiel 15

4 g des Prepolymers A wurden mit einer Mischung aus 0,55 g Aspartat B und 3,45 g eines Polyesterpolyols mit einem Ethylenoxidanteil von 52 % und einem Propylenoxidanteil von 35 % und der Funktionalität 3 (3460 mPas/25°C) in einem Becher gut verrührt und auf das zu klebende Gewebe dünn aufgetragen. Es hat nach 3 min eine mittelmäßige, nach 6 min eine gute Klebung stattgefunden. Durch Zug konnten die Gewebestücke mit leichter Faserschädigung voneinander getrennt werden. Bei Auftragung auf die Oberfläche des Gewebes oder auf Haut fand in einem Zeitraum von 10 Minuten keine vollständige Aushärtung statt.

### Beispiel 16

4 g des Prepolymers A wurden mit einer Mischung aus 0,55 g Aspartat B und 3,45 g eines Polyethers des Molekulargewichts 3005 mit einem Ethylenoxidanteil von 55 % und einem Propylenoxidanteil von 45 % und der Funktionalität 3 (550 mPas/25°C) in einem Becher gut verrührt und auf das zu klebende Gewebe dünn aufgetragen. Es hat nach 3 min eine starke Klebung stattgefunden. Durch Zug konnten die Gewebestücke nicht ohne Faserschädigung voneinander getrennt werden. Bei Auftragung auf die Oberfläche des Gewebes oder auf Haut fand in einem Zeitraum von 10 Minuten keine vollständige Aushärtung statt.

### Beispiel 17

4 g des Prepolymers A wurden mit einer Mischung aus 0,55 g Aspartat B und 3,45 g eines Polyethers des Molekulargewichts 673 mit einem Propylenoxidanteil von 3,6 %, einem Ethylenoxidanteil von 96,4 % und der Funktionalität 3 (700 mPas/25°C) in einem Becher gut verrührt und auf das zu klebende Gewebe dünn aufgetragen. Es hat nach 2 min eine gute Klebung stattgefunden. Durch Zug konnten die Gewebestücke nicht ohne Faserschädigung voneinander getrennt werden. Bei Auftragung auf die Oberfläche des Gewebes oder auf Haut fand in einem Zeitraum von 5 Minuten keine vollständige Aushärtung statt.

### Beispiel 18

4 g des Prepolymers A wurden mit einer Mischung aus 0,55 g Aspartat B und 3,45 g eines Polyethers des Molekulargewichts 4549 mit einem Propylenoxidanteil von 27,3 %, einem Ethylenoxidanteil von 72,7 % und der Funktionalität 3 (1070 mPas/25°C) in einem Becher gut verrührt und auf das zu klebende Gewebe dünn aufgetragen. Es hat nach 2 min eine mittelmäßige Klebung stattgefunden. Durch Zug konnten die Gewebestücke nicht ohne Faserschädigung voneinander getrennt werden. Bei Auftragung auf die Oberfläche des Gewebes oder auf Haut fand in einem Zeitraum von 10 Minuten keine vollständige Aushärtung statt.

### Beispiel 19 in vitro Klebung von Muskelgewebe

4 g des Prepolymers A wurden mit einer Mischung aus 0,45 g PEG 500 Dimethylether (19 mPas/25°C) und 0,55 g Aspartat B in einem Becher gut verrührt und auf das zu klebende Gewebe dünn aufgetragen. Es hat nach 2 min eine starke Klebung stattgefunden. Durch Zug konnten die Gewebestücke nicht ohne Faserschädigung voneinander getrennt werden. Bei Auftragung auf die Oberfläche des Gewebes fand eine vollständige Aushärtung unter Bildung eines durchsichtigen Filmes innerhalb von 5 Minuten statt.

### Beispiel 20 in vitro Klebung von Muskelgewebe

4 g des Prepolymers A wurden mit einer Mischung aus 3,45 g PEG 500 Dimethylether (19 mPas/25°C) und 0,55 g Aspartat B in einem Becher gut verrührt und auf das zu klebende Gewebe dünn aufgetragen. Es hat nach 5 min nur eine schwache Klebung stattgefunden. Bei Auftragung auf die Oberfläche des Gewebes fand keine Aushärtung innerhalb von 10 Minuten statt.

## Patentansprüche

1. Klebstoffsysteme umfassend
A) isocyanatfunktionelle Prepolymere erhältlich aus
A1) aliphatischen Isocyanaten und
A2) Polyolen mit zahlenmittleren Molekulargewichten von ≥ 400 g/mol und mittleren OH-Funktionalitäten von 2 bis 6
und
B) eine Härterkomponente umfassend
B1) aminofunktionelle Asparaginsäureester der allgemeinen Formel (I) 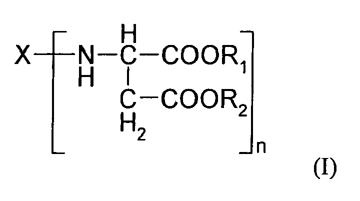 wobei
X ein n-wertiger organischer Rest ist, der durch Entfernung der primären Aminogruppen eines n-wertigen Amins erhalten wird,
R₁ , R₂ gleiche oder verschiedene organische Reste sind, die keinen Zerewitinoff-aktiven Wasserstoff aufweisen und
n eine ganze Zahl von mindestens 2 ist
und
B2) organische Füllstoffe mit einer nach DIN 53019 gemessenen Viskosität bei 23°C im Bereich von 10 bis 6000 mPas
und
C) gegebenenfalls Umsetzungsprodukte von isocyanatfunktionellen Prepolymeren gemäß der Definition der Komponente A) mit Asparaginsäureestern gemäß Komponente B1) und/oder organischen Füllstoffen gemäß Komponente B2).

2. Klebstoffsysteme gemäß Anspruch 1, **dadurch gekennzeichnet, dass** die in A2) eingesetzten Polyole zahlenmittlere Molekulargewichte von 4000 bis 8500 g/mol aufweisen.

3. Klebstoffsysteme gemäß Anspruch 1 oder 2, **dadurch gekennzeichnet, dass** die in A2) Polyalkylenoxid-Polyether eingesetzt werden.

4. Klebstoffsysteme gemäß einem der Ansprüche 1 bis 3, **dadurch gekennzeichnet, dass** als organische Füllstoffe der Komponente B2) Polyetherpolyole eingesetzt werden.

5. Klebstoffsysteme gemäß einem der Ansprüche 1 bis 4, **dadurch gekennzeichnet, dass** es sich um einen Gewebekleber für menschliches oder tierisches Gewebe handelt.

6. Verfahren zur Herstellung von Klebstoffsystemen gemäß einem der Ansprüche 1 bis 5, bei dem die Komponenten A) und B) gegebenenfalls C) in einem Verhältnis von NCO-reaktiven Gruppen zu freien NCO-Gruppen von 1:1,5 bis 1:1 1 miteinander vermischt werden.

7. Klebstoffsysteme erhältlich nach Verfahren gemäß Anspruch 6.

8. Verfahren zum Verschließen oder Verbinden von Zellgeweben, **dadurch gekennzeichnet, dass** Klebstoffsysteme gemäß einem der Ansprüche 1 bis 5 oder 7 eingesetzt werden.

9. Verwendung von Klebstoffsystemen gemäß einem der Ansprüche 1 bis 5 oder 7 zur Herstellung eines Mittels zum Verschließen oder Verbinden von Zellgeweben.

10. Klebstofffilme und Verbundteile erhältlich unter Verwendung von Klebstoffsystemen gemäß einem der Ansprüche 1 bis 5 oder 7.

11. 2-Kammerdosiersystem umfassend ein Klebstoffsystem gemäß einem der Ansprüche 1 bis 5 oder 7, wobei eine Kammer das Prepolymer der Komponente A) und die andere die Härterkomponente B) und gegebenenfalls C) umfasst.
